# EUROPEAN PATENT APPLICATION

(11) **EP 4 461 129 A1**
(43) Date of publication of application: **13.11.2024**
(21) Application number: 23172320.6
(22) Date of filing: 09.05.2023
(51) Int. Cl.: A01H 4/00, C12P 7/22, A01H 5/08, A01H 6/52

(54) **EDIBLE PLANT CELL CULTURE BIOMASS BASED ON FRUIT ENDOCARP AND/OR SEED EXPLANTS AND METHODS OF ITS PREPARATION**

(71) Applicant: ZHAW - Zürcher Hochschule für Angewandte Wissenschaften, 8820 Wädenswil (CH)
(72) Inventor: EIBL, Regine, Wädenswil (CH); EIBL, Dieter, Wädenswil (CH); HÜHN, Tilo, Wädenswil (CH); MOZAFFARI, Fruhar, Wädenswil (CH)
(74) Representative: Grünecker Patent- und Rechtsanwälte PartG mbB

(57) **Abstract**

A method of processing plant cell cultures based on fruit explants is described, comprising the steps of: a) inducing callus from an explant of a fruit selected from drupes, nuts, legumes or berries on callus induction medium and propagating the callus on callus growth medium; b) selecting a callus clone from the grown callus culture and initiating a suspension cell culture comprising dedifferentiated cells by inoculating the selected callus clone into a suspension cell culture medium; c) growing fruit suspension cells for a time and under conditions sufficient to produce a suspension including a biomass comprising secondary metabolites; and d) separating the biomass from the suspension to provide a liquid phase and a solid, in vitro-manufactured biomass and optionally extracting secondary metabolites from the *in vitro*-manufactured biomass to provide a secondary metabolite extract, wherein the fruit is selected from and the explant contains endocarp and/or seed material from the drupes, nuts, legumes or berries. The method enables biomass to be manufactured *in vitro* at improved efficiencies and yields. In addition, in vitro-manufactured biomass prepared by the aforementioned method, uses of the same, and related food compositions are described.

## Description

### FIELD OF INVENTION

This invention relates to methods and/or techniques for the *in vitro-*production of biomass from plant cell cultures on the basis of endocarp and/or seed explants from fruits.

In certain embodiments, this invention relates to use of said *in vitro*-manufactured biomass in wet or powder form as food, food additive or in cosmetic or pharmaceutical preparations.

In addition, food compositions obtained by said methods and/or techniques are disclosed.

### BACKGROUND OF THE INVENTION

In the recent decades, various methods based on plant cell culture (PCC) technology have been developed for the isolation of secondary metabolites for the use in food supplements, pharmaceutical compositions and for cosmetic applications (see e.g. R. Eibl et al., Applied Microbiology and Biotechnology 2018, 102, 8661-8675).

PCC technology is generally considered as a promising alternative to secondary metabolite production to avoid the disadvantages of conventional agricultural methods, which are often associated with excessive water consumption, high carbon footprint, seasonal dependence and the use of pesticides and herbicides.

Especially polyphenolic compounds, such as procyanidins, have received widespread attention as target secondary metabolites. For example, EP 2 414 509 B1 discloses the PCC-based manufacture of alkaloid-free polyphenol preparations, which involves growing callus from an immature *Theobroma* or *Herrania* floral explant selected from staminode, sepal end petal based explants, leaves, stem, meristem, nodes or internodes, selecting a rapidly growing cell line from the callus culture, initiating a cell suspension culture by inoculating the rapidly growing cell line into liquid medium, growing cells in the suspension culture to result in production of cocoa procyanidins, and harvesting cocoa procyanidins from the cell suspension culture.

However, it would be desirable to provide efficient methods for preparing cell suspension cultures for various other plants (such as drupes, nuts, legumes or berries, for example), each of which may have their own characteristic set of valuable, physiologically active secondary metabolites.

For example, parts of the avocado plant (*Persea americana*), a member of *Lauraceae* family that grows in many tropical and subtropical regions, finds widespread use as food and also has promising properties for medical applications (see A. N. F. Abubakar et al., Asian Pacific Journal of Tropical Biomedicine 2017, 7 (5), 397-400). The seed is reported to contain secondary metabolites that belong to the class of alkaloids, triterpenoids, tannins, flavonoids, saponins, and polyphenols, which exhibit excellent antioxidant properties (see W. Wang et al., Food Chem. 2010, 122, 1193-1198). In addition, due to its advantageous contents of dietary fibers and textural properties, avocado seed powder may be used in functional food design (see M. Siol, A. Sadowska, Agriculture 2023, 13, 316).

However, avocados have a significant demand for irrigation water. Specifically, it is reported in M. M. Mekonnen, A. Y. Hoekstra, Hydrol. Earth Syst. Sci. 2011, 15, 1577-1600 that the global average water footprint for avocados amounts to 1,981 m³/t (grey, blue and green water in total). Hence, the provision of an effective PCC-based preparation method for avocado seed powder would be highly desirable, since water consumption required for plant cell cultivation is only a fraction of the water demand for conventional agricultural cultivation (i.e. less than 100 m³/t and typically less than 50 m³/t).

Nevertheless, successful mass propagation of suspension cell cultures at a scale suitable for the use in pharmaceutical, food and cosmetic applications remains highly challenging.

For instance, it has been found that the use of plant meristems (such as shoot apical system root apical system, or cambium) or leaves for the preparation of explants is often accompanied by a low rate of success in callus induction, which substantially impedes the preparation of cell cultures in terms of process efficiency and yield.

Accordingly, the provision of methods and products that overcome the above disadvantages is desirable.

### SUMMARY OF THE INVENTION

The present invention solves this object with the subject matter of the claims as defined herein. The advantages of the present invention will be further explained in detail in the section below and further advantages will become apparent to the skilled artisan upon consideration of the invention disclosure.

Generally speaking, the present invention provides a method of processing plant cell cultures based on fruit explants, comprising the steps of: a) inducing callus from an explant of a fruit selected from drupes, nuts, legumes or berries on callus induction medium and propagating the callus on callus growth medium; b) selecting a callus clone from the grown callus culture and initiating a suspension cell culture comprising dedifferentiated cells by inoculating the selected callus clone into a suspension cell culture medium; c) growing fruit suspension cells for a time and under conditions sufficient to produce a suspension including a biomass comprising secondary metabolites; and d) separating the biomass from the suspension to provide a liquid phase and a solid, *in vitro*-manufactured biomass and optionally extracting secondary metabolites from the *in vitro*-manufactured biomass to provide a secondary metabolite extract; wherein the fruit is selected from and the explant contains endocarp and/or seed material from the drupes, nuts, legumes or berries. Advantageously, the method of the present invention enhances the success rate of callus induction and enables improved cell culture growth efficiency.

In a further aspect, the present invention relates to *in vitro*-manufactured biomass obtained by the aforementioned method.

In another aspect, the present invention relates to the use of said *in vitro-*manufactured biomass secondary metabolite extract as food, food additive or in cosmetic or pharmaceutical preparations.

In a further aspect, the present invention relates to a food composition, containing a mixture of: *in vitro*-manufactured biomass or secondary metabolite extract as described above in an amount of 25% to 99% by weight of the total weight of the food composition, and one or more edible ingredients other than the *in vitro*-manufactured biomass in an amount of 1 % to 75% by weight of the total weight of the food composition.

### BRIEF DESCRIPTION OF THE DRAWINGS

FIG. 1 schematically illustrates exemplary methods of processing plant cell cultures according to the first embodiment of the present invention.
FIG. 2A depicts a primary callus from the avocado pit (origin: Dominican Republic) after three weeks.
FIG. 2B depicts a primary callus from the avocado pit (origin: Israel) after the 8^{th} passage.

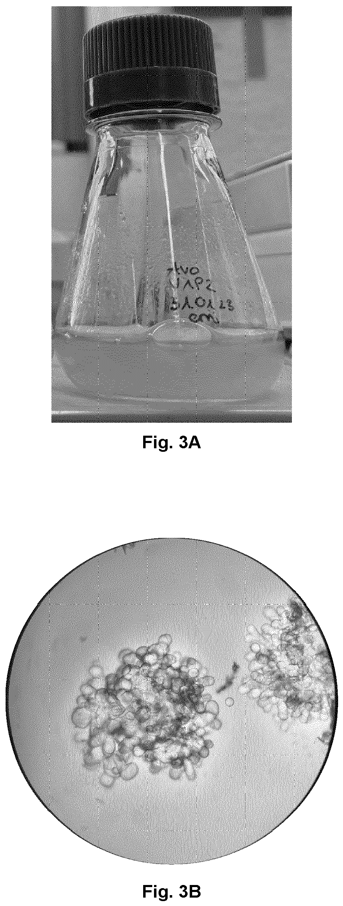

FIG. 3A shows the maintenance culture in a shake flask.
FIG. 3B shows a microscopic image (10-fold magnification) of the cell cultures in the shake flask of Fig. 3A.
FIG. 4 shows is a photograph of a guacamole sample produced according to the present invention.

### DETAILED DESCRIPTION OF THE INVENTION

For a more complete understanding of the present invention, reference is now made to the following description of the illustrative embodiments thereof:

### Methods of Processing Plant Cell Cultures

In a first embodiment, the present invention relates to a method of plant cell cultures based on fruit explants, comprising the steps of: a) inducing callus from an explant of a fruit selected from drupes, nuts, legumes or berries on callus induction medium and propagating the callus on callus growth medium; b) selecting a callus clone from the grown callus culture and initiating a suspension cell culture comprising dedifferentiated cells by inoculating the selected callus clone into a suspension cell culture medium; c) growing fruit suspension cells for a time and under conditions sufficient to produce a suspension including a biomass comprising secondary metabolites; and d) separating the biomass from the suspension to provide a liquid phase and a solid, *in vitro*-manufactured biomass and optionally extracting secondary metabolites from the *in vitro*-manufactured biomass to provide a secondary metabolite extract, wherein the fruit is selected from and the explant contains endocarp and/or seed material from the drupes, nuts, legumes or berries.

Accordingly, the present invention is not based on the cultivation of callus cell cultures from immature floral explants (e.g. petals, sepals, or staminodes) or shell (exocarp) or pulp (mesocarp) material, but uses endocarp and/or seed material of the fruits to establish the production cell line, which surprisingly improves the success rate in the induction process.

It is emphasized that the term "fruit", as used herein, denotes fruits in the botanical sense, which may be defined as the seed-bearing structure in flowering plants that is formed from the ovary after flowering. Notably, such a definition may also include species labelled as vegetables in the culinary sense.

Legumes, as defined herein, relate to fruits of plants in the pea family (*Fabaceae*) and may include, but are not limited to soybeans, peanuts, navy beans, kidney beans, lima beans, string beans, pinto beans, chickpeas, lentils, peas, black-eyed peas, for example. As non-limiting examples of nuts, almonds, pecans, walnuts, cashews, pistachios, peanuts, kola nuts, palm nuts, hazelnuts, filberts, Brazil nuts, macadamia nuts and chestnuts may be mentioned. Drupes and berries both relate to fleshy fruits, wherein drupes are characterized by having a fleshy mesocarp but a hardened, lignified endocarp surrounding the seed (kernel), and berries are characterized by having a fleshy endocarp, as well as mesocarp, and may have more than one seed. Exemplary drupes for the purpose of the present invention include apricots, olives, dates, cherries, blackberries, peaches, coconuts and mangoes, for example. Examples of berries include, but are not limited to avocado, banana, barberry, grape, bearberry, blueberry, cranberry, lingonberry, crowberry, coffee cherry, currant, tomato, goji berry, elderberry, khaki, lychee, pumpkin, cucumber and watermelon. In a preferred embodiment, the fruit is selected from berries, especially preferably from the family *Lauraceae,* elderberry, khaki, blueberry, blackcurrant, lychee, raspberry, grapes and dates. Even further preferably, the fruit is avocado (*Persea americana*).

The endocarp is the tissue layer immediately adjacent to the seed and provides a physical barrier, protecting the seed from disease and herbivory. In a preferred embodiment, the explant contains both endocarp and seed material from the fruits. In general, in case of fruits with a woody, lignified endocarp, such as most nuts and drupes, it is preferable to predominantly or exclusively use seed (kernel) material as the explant in step a). In case of berries, it is preferred to use both endocarp and seed material.

In a preferred embodiment, cocoa beans are excluded from the term "seeds" and cocoa pods are excluded from the term "fruits" according to the present invention, respectively.

In view of a public reluctance to accept genetically modified food sources, the cultivated cell cultures are preferably not genetically modified. Moreover, it is further preferred that any metabolic engineering applied in the present process is performed without genetic modification technology. Beside of the aspiration to preserve the natural state of the plant source and to simulate natural biotransformation as closely as possible, such an approach also favorably reduces the regulatory hurdles for commercialization.

While the maturity degree of the fruits is not specifically limited, the use of fruits identified as ripe by colour and manual inspection may be preferred.

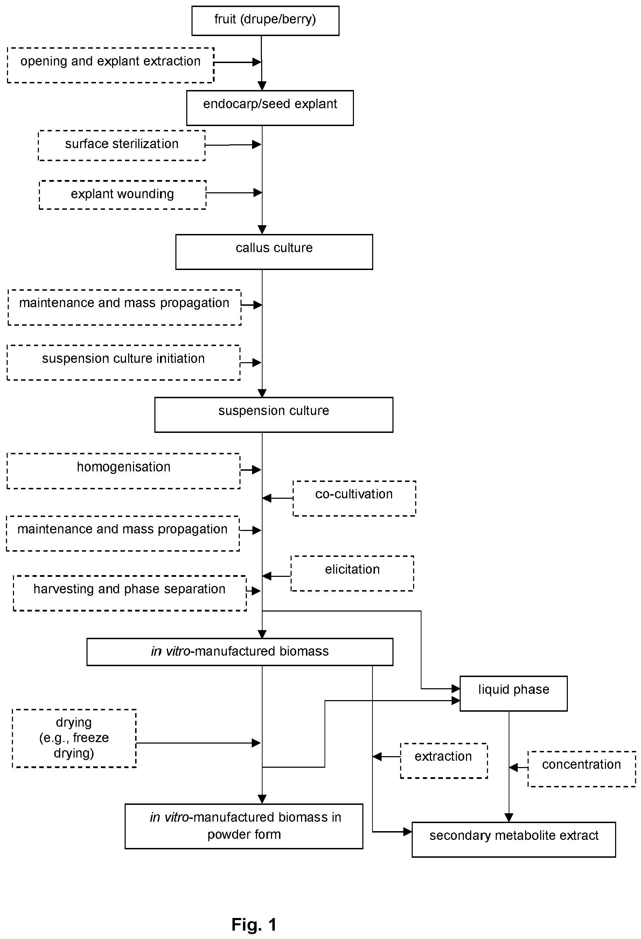

An exemplary method according to the first embodiment of the invention, as will be explained in detail below, is illustrated in Fig. 1. However, it is understood that the methods are not limited to the depicted process.

The subject process typically begins with opening or cutting open a washed and/or surface-sterilised fruit under aseptic conditions, which is preferably followed by a step of carefully removing exocarp (shell or outer hull) and mesocarp (flesh or pulp).

The washing and/or surface sterilisation steps are not particularly limited as long as they effectively reduce microbial surface contamination, and suitable methods may include chemical sterilisation (e.g., with ethanol solution (≥ 70% (v/v)), sodium hypochlorite solution (e.g. 5% (v/v) or the like), washing in sterile water, surface sterilisation or pasteurization by means of heat and/or irradiation with ionizing rays (e.g. UV irradiation), ultrasound-based sterilisation and combinations thereof.

In step a), formation of callus, a mass of unorganized, undifferentiated cells is induced by wounding of the explant (e.g. by cutting) and incubating the same onto a callus induction medium.

The callus induction medium is not especially limited and typically represents a semisolid or preferably solid tissue culture medium comprising the ions Ca²⁺, SO₄²⁻, Mg²⁺, PO₄³⁻, Cl⁻, NO₃⁻ and NH₄⁺. Examples thereof include, but are not limited to Murashige and Skoog (MS) medium (Murashige and Skoog, Physiol. Plant 1962, 15, 473-497), WPM medium (Lloyd and McCown, Int. Plant Prop. Soc. Proc. 1980, 30, 421), DKW basal medium (Driver and Kuniyuki, Hortsci. 1984, 19, 507-509), Gamborg B5 medium (Gamborg, Miller Ojima, Exp. Cell res. 1968, 50, 151) or similar plant tissue culture (PTC) media and modifications thereof.

The callus induction medium typically further contains carbohydrates (such as glucose, sucrose or saccharose) as a carbon source. In preferred embodiments, the concentration of the carbon source in the (fresh) callus induction medium is at least 10 g/l, more preferably higher than 15 g/l, further preferably between 18 and 40 g/L and especially preferably between 20 and 35 g/l.

In order to mitigate the costs associated with the extensive use of MS media or the like, the cell culture medium may be preferably produced by a method comprising: 1) growing microgreens from edible seeds of vegetables, cereals or herbs; 2) harvesting the microgreens in an immature state; 3) grinding the harvested microgreens to an average particle size of 500 µm or less (preferably 100 µm or less) to provide a mash, preferably at temperatures below 65°C, such as below 40°C, or at 0 to 35°C; 4) subjecting the ground mash to a phase separation resulting in a solid phase and a liquid phase; 5) optionally adding water to dilute the liquid phase; and 6) sterilising the optionally diluted liquid phase to provide the cell culture medium. As an alternative to steps 5) and 6), the liquid phase may be subjected to a drying and/or concentration step to provide a supplement, which may be added to a conventional cell culture medium (e.g. as a carbon source). In a further preferred embodiment of said method, step 1) may comprise growing microgreens of corn (*Zea mays*)*,* preferably sweet corn (*Zea mays convar. saccharata var. rugosa*), under dark and humid conditions.

In an alternatively preferred embodiment, the cell culture medium may be prepared by a method comprising the steps of: 1) providing a starch-containing plant (preferably selected from crop plants, preferably from cereals, legumes, oil crop, sugarcane, sugar beet, root vegetables and fruits, more preferably from corn, wheat, legumes, sugarcane, sugar beet, carrots, potatoes, sweet potatoes and fruits); 2) grinding the starch-containing plant to a mean particle size of 500 µm or less to provide a mash; 3) subjecting the ground mash to a phase separation resulting in a solid phase and at least one liquid phase; 4) separately processing the solid phase and the at least one liquid phase, including subjecting the separated solid phase to a saccharification step (preferably including an enzymatic treatment, acidic treatment, solid catalyst-assisted microwave irradiation, thermal treatment under pressure, steam treatment or combinations thereof); 5) recombining the separately processed phases; and 6) adding water to the recombined phases to provide the cell culture medium. As an alternative to step 6), the liquid phase obtained in step 4) or the recombined phases obtained in step 5) may be subjected to a drying and/or concentration step to provide a supplement, which may be added to a conventional cell culture medium (e.g. as a carbon source).

The fruit explants are preferably cultured on the callus induction medium at temperatures of 25 ± 5° C for a period of 5 to 35 days, such as from 10 to 20 days.

Subsequently, the induced callus is transferred and propagated on callus growth medium.

The callus growth medium may contain higher levels of carbohydrates (such as glucose, sucrose or saccharose) as a carbon source than the callus induction medium to support growth and production of secondary metabolites. In preferred embodiments, the concentration of the carbon source in the fresh callus growth medium is at least 25 g/l, more preferably higher than 30 g/l, further preferably at least 40 g/l and especially preferably between 45 and 75 g/l.

In preferred embodiments, the callus induction and/or callus growth media are further supplemented with one or more vitamins (e.g. Vitamin B5) and/or one or more growth regulators (e.g., sterile coconut water, a cytokinin (such as thidiazuron, zeatin or kinetin), gibberellin (e.g., gibberellic acid) or an auxin (e.g., 2,4-dichlorophenoxyacetic acid (2,4-D), indoleacetic acid (IAA) or indolebutyric acid (IBA)) or preferably a combination thereof. Phytohormones present in or extracted from meristem plant cells, preferably those of the plant bearing the fruits for the purpose of the present invention may be preferably used, which also enable induction and growth control without addition of chemically synthesized growth regulators. In the case of the growth medium, it is important to appropriately select the growth regulators so as to avoid any negative effects on the consumer during the later application of the cell culture. For instance, for the manufacture of a food product, it is important to select food-compliant growth regulators for the callus growth medium.

Callus cultures may be preferably maintained in fresh callus growth medium by periodically transferring at least a portion of the callus to a fresh medium under aseptic conditions, typically in a biomass/medium weight ratio of between 20:80 to 40:60 (subcultivation). Suitable subcultivaton intervals (typically around 3 to 4 weeks) may be determined by the skilled artisan depending on the growth performance and the decrease of carbohydrate and nitrogen concentration in the medium (e.g. by periodical sampling and HPLC analysis).

Notably, step a) may include further substeps performed in series (e.g. subcultivation, transfer to multiple types of callus induction media and/or multiple types of callus growth media) or in parallel (e.g. combination of different induced calli for the growth phase).

Once sufficient callus mass has been established, a well-growing and producing callus line is selected and a suspension cell culture comprising dedifferentiated cells is initiated by inoculating the selected callus mass into a liquid medium supporting cell growth and the production of secondary metabolites of interest in step b).

Optionally, the selected calli may be first transferred or pooled into shake flasks containing liquid culture medium and shaken to promote homogenisation during step b1) or before being transferred to a bioreactor to step c).

The suspension cell culture medium used in step b) and/or c) is not particularly limited and may be a liquid variant of the callus growth medium described above.

In general, it is preferred that the suspension cell culture medium at least comprises a plurality of mineral salts, further preferably in combination at least one or more growth regulators (e.g., phytohormones) and at least one carbohydrate as carbon source.

Preferably, the concentration of a carbohydrate (such as glucose, sucrose or saccharose) in the fresh suspension cell culture medium is at least 25 g/l, more preferably at least 30 g/l, further preferably at least 40 g/l and especially preferably between 45 and 75 g/l. Working within these ranges was shown to enable an unexpectedly high increase of biomass growth.

Suspension cultures may subcultivated by periodically transferring at least a portion of the cultured cells to a fresh medium under aseptic conditions, typically in a biomass/medium weight ratio of between 20:80 to 40:60. Typical subcultivaton intervals range from around 14 to 21 days, and may be suitably adjusted by the skilled artisan upon monitoring the cell growth performance and the decrease of carbohydrate and nitrogen concentration in the suspension medium.

For step c), the suspended cultures are transferred into the bioreactor and grown for a time and under conditions sufficient to produce a biomass comprising secondary metabolites.

According to the present invention, the bioreactor used in step c) is selected from a wave-mixed bioreactor, a stirred tank bioreactor, an orbitally shaken bioreactor, a bubble column bioreactor or an air-lift bioreactor, preferably a bubble column bioreactor or an air-lift bioreactor. Notably, although the latter reactor types are commonly known, especially for fermentation purposes (e.g. in beverage production), their successful application for the growth of suspension cells is all but trivial. For instance, a rigid cell wall and extensive vacuoles are known to make plant cells sensitive to shear stress, so that - contrary to fermentation systems - an upscaling of plant cell culture production via stirred tank bioreactors is often limited and/or hindered by cell death induced by the impeller. In general, several difficulties are typically associated with the exchange and upscaling of bioreactor technology, which mainly involve reduced yield, small growth rates and product release, underexpression of desirable traits and poor culture stability. Advantageously, it has been found that wave-mixed bioreactors, stirred tank bioreactors, orbitally shaken bioreactors, bubble column bioreactors and air-lift bioreactors may be effectively operated at a scale required for cost-effective processing of food and simultaneously enable favorable control of hydrodynamic parameters and aeration conditions to ensure excellent and consistent quality of the *in* vitro-manufactured biomass.

In addition, it has been surprisingly found that the yield of secondary metabolites, such as polyphenolic compounds, for example is sensitive to the selection of the bioreactor type.

In preferred embodiments, the bioreactor has a volume of at least 0.05 m³, more preferably at least 0.1 m³, further preferably at least 1 m³, and especially preferably at least 5 m³, such as 10 m³ or more, 100 m³ or more, 150 m³ or more, or even 200 m³ or more.

Wave-mixed bioreactors may be preferable if high cell growth rates and biomass productivity are given preference over upscaleability.

Bubble column bioreactors or air-lift bioreactors are particularly preferred as they may be simultaneously operated at the aforementioned high scales, built with larger working volumes than stirred tank bioreactors, allow oxygen mass transfer at low power input levels, and are inexpensive in terms of investment and maintenance, which is a crucial factor for cost-effective production of plant cell cultures at large scale.

If a stirred tank bioreactor is used, designs using an end-face mechanical seal or a magnetic drive with free levitating impeller are preferably employed, since they are almost maintenance-free and reduce risk of contamination. The type of impeller and its dimensions may be suitably selected by the skilled artisan depending on the mixing time, shear gradient, specific power input and oxygen transfer rate. Multi-stage configurations combining axial and radial flow impellers may also be employed.

In a preferred embodiment, the bioreactor is equipped with an air recirculation system. By operating the bioreactor (especially the airlift and bubble column reactors) so that air bubbles transported through the cell suspensions are recirculated, the oxygen flow rate may be controlled at desirably low levels without imparting the mixing operation, while aromatic volatiles are retained or re-fed in the system, which may be utilized to fine-tune the aromatic profile of the cell culture biomass. Simultaneously, air recirculation reduces the amount of sterilised air necessary for bioreactor operation and mixing, which may substantially lower the manufacturing costs, especially in large-scale production.

In preferred embodiments, hydrodynamic parameters and aeration conditions of the bioreactor are determined based on the concentration of target secondary metabolites present in the biomass obtained in step d).

In general, the term "secondary metabolites", as used herein, denotes compounds that are not required for the growth or reproduction of the fruit-bearing plant but are produced to confer a selective advantage to the same. While not being limited thereto, secondary metabolites comprise one or more phenolic compounds or polyphenols. In further preferred embodiments, the target secondary metabolites selected to control hydrodynamic parameters and aeration conditions comprise one or more selected from the group of polyphenols, vitamins (e.g., vitamin A, vitamin B1, vitamin B2, vitamin B3, vitamin C, and vitamin E), and aromatic compounds, which may be characteristic to the fruit used. For example, an overview of secondary metabolites produced during the natural development of avocado seeds and which may be used as target secondary metabilites is disclosed in M. Sio., A. Sadowska, Agriculture 2023, 13, 316, and will typically include phenolic compounds or polyphenols selected from gallic acid, chlorogenic acid, p-hydrobenzoic acid, benzoic acid, coffee acid, catechin, epigallocatechin, glycoside-3-O-kaempferol, quercetin, lutein and rutinoside-3-O-quercetin.

The concentrations of individual target secondary metabolites may be monitored (by methods known in the art, including HPLC-MS or GC-MS) during the cultivation phase and hydrodynamic parameters or aeration conditions may be adjusted if the measured concentration deviates form a predetermined target value.

It is noted that the yield of secondary metabolites is highly sensitive to changes in type, as well as hydrodynamic parameters and aeration conditions of the bioreactor (particularly mixing time, specific power input and/or volumetric oxygen transfer coefficient). Accordingly, their adjustment enables tuning and consistent control of the desired sensory properties (including the aromatic profile) and texture of the resulting *in vitro*-manufactured biomass.

The specific power input is typically adjusted within a range of 50 to 900 W/m³, preferably between 80 and 850 W/m³, and further preferably between 100 and 800 W/m³. It has been surprisingly found that the secondary metabolite content (e.g., content of polyphenols), aroma and flavor profile and of the resulting cell culture biomass may be varied in a simple manner by operating in different specific power input regimes within the above-identified range.

The volumetric oxygen transfer coefficient is preferably adjusted within a range of 0.3 to 80 h⁻¹, more preferably in a range of from 1 to 75 h⁻¹, especially preferably in a range of 5 to 50 h⁻¹. In case of airlift and bubble column reactors, the volumetric oxygen transfer coefficient may be higher, e.g. in the range of 0.3 to 180 h⁻¹, preferably in a range of 1 to 120 h⁻¹, more preferably in a range of 5 to 100 h⁻¹. However, when operating the latter reactors with an air recirculation system, a range of 0.3 to 80 h⁻¹ may be preferable.

By monitoring the concentration of the abovementioned secondary metabolites, the mixing time may be further adjusted to ensure optimized yield.

Beside of the control of hydrodynamic and aeration parameters, step c) may involve further sub-steps to promote the growth rate, stimulate production of secondary metabolites and control the sensory properties of the resulting biomass.

For instance, one or more homogenisation steps may be incorporated into step c) to remove floating particles and avoid formation of larger cell aggregates. While methods of homogenisation are not particularly limited, gentle homogenisation procedures such as sedimentation and/or filtration are preferred to ensure that the average size of cell aggregates does not exceed 500 µm, which increases the specific growth surface and thus promotes biomass growth velocity without damaging cell components sensitive to heat, pressure and/or mechanical stress.

In addition, multiple-stage cultures may be considered to further promote the development of secondary metabolites in order to account for the tendency that several developmental stages are required for production of the non-growth associated compounds.

In another preferred embodiment, step c) comprises co-cultivating the suspension cells with microorganisms. The term "microorganism", as used herein, is understood to encompass microscopic organisms, which may exist in its single-celled form or a colony of cells, including bacteria and single-cellular fungi or algae. Microorganisms may be selected from one or more species from the group of yeasts, lactic acid bacteria and/or acetic acid bacteria, for example.

Step c) preferably also comprises an elicitation step comprising the addition of one or more abiotic or biotic elicitors to stimulate biosynthesis of secondary metabolites, to reduce the production of undesirable substances and/or to reduce the process time necessary to reach a high product concentration. Further, elicitation may result in the formation of novel compounds. Synergistic effects using multiple simultaneous elicitation treatments or sequential elicitor protocols may also be envisaged.

As examples of biotic elicitors, plant hormones (e.g. salicylic acid and its derivatives and analogs), microorganism-derived elicitors (e.g. microbial pathogens, bacterial extracts, bacteria- and fungi-derived peptides and proteins) and plant cell wall fragments (e.g. chitosan, chinin, oligosaccharins) may be mentioned.

Abiotic elicitation includes abiotic stress used to elicit the production of defense chemicals (e.g. phytoalexins), e.g. by variation of pressure and/or temperature; addition of natural agents extracted from other plants; addition of chemical compounds, such as AgNOs, AlCl₃, CaCl₂, CuCl₂, KCI, zinc ions or ozone; addition of pathogen-derived compounds, pesticides, herbicides, fungicides, or bactericides; limitation of sources of phosphorus, nitrogen and/or carbon; water limitation; exposure to high salt concentrations; and exposure to UV-light, microwaves, ultrasonic waves, radiofrequency, infrared radiation, or ionizing radiation.

In a particularly preferred embodiment of abiotic elicitation, growth of the biomass in step c) is enhanced by exposing the suspension cell culture to artificial light having a wavelength in the range of 300 to 700 nm and with an intensity sufficient to promote biomass growth and/or by removal of a fraction of the liquid medium during the growth phase.

It is also preferred that step c) comprises the addition of enzymes, phytohormones or biosynthetic precursors to the medium. Biosynthetic precursors include aroma precursors naturally produced in the food source used as starting material or produced in another species, or amino acids (such as glutamine, glycine, and serine), for example.

Once a cell culture has reached the desired concentration and/or composition of target secondary metabolites, the cells are harvested and the biomass is separated from the suspension in step d) to provide *in vitro*-manufactured biomass and a liquid phase.

The separation method is not particularly limited and may include one or more solid-liquid separation steps, such as filtration, sedimentation, pressing (e.g., screen or screw pressing) decanting or centrifugation under sterile conditions, for example.

In preferred embodiments, the solid phase is rinsed and washed in sterile water to eliminate flavor artifacts originating from the culture medium.

Optionally, the solid phase may be subjected to a grinding step (e.g., to an average particle diameter of 300 µm or less, 200 µm or less, or 100 µm or less) in order to alter the texture of the resulting biomass, preferably in terms of single or multiple wet grinding step(s) in the culture medium before solid-liquid separation or upon adding sterile water or an alternative water-containing liquid to the separated solid phase. At this stage, additional flavors may be introduced with the use of said alternative water-containing liquid. Grinding may be performed by using disc mills (e.g. a perforated disc mill), colloid mills (e.g. toothed colloid mills), or corundum stone mills, for example.

In order to provide the product in powder form, the thus obtained biomass, which may be initially present in form of a slurry (having a dry matter content of at least 60% by weight of the slurry, preferably at least 70% by weight of the slurry), is preferably subjected to drying prior to packaging, storage and/or further processing, e.g. by drum drying, rotary drying or freeze-drying, among which freeze-drying is especially preferred. However, it is understood that no dedicated drying step is necessary if the biomass is immediately processed (e.g. to food pastes etc.).

Notably, solvent extraction may be employed to provide a secondary metabolite concentrate from the solid phase or biomass, respectively. In this case, it is preferred to use aqueous organic solvents comprising ethanol, methanol, acetone, isopropyl alcohol, ethylacetate or combinations thereof, optionally in combination with an acid (e.g., ascorbic acid). The solvent may be then removed by one or more concentration steps to provide secondary metabolite extract. In order to minimize degradation of heat-sensitive secondary metabolites (e.g. polyphenols), concentration methods at low temperatures (≤ 45°C) and reduced pressure are preferred.

The biomass may also be subjected to post-processing steps to selectively separate flavors and aromatic compounds. Such processing steps may include extraction methods commonly used in the art, including solvent extraction and thermal treatment (effecting an evaporation of flavors), for example.

The liquid phase obtained after solid-liquid separation may also contain desirable water-soluble flavors and especially antioxidants, which may be optionally recovered by subjecting the liquid phase to a concentration step. Enhancement of flavors may be achieved using reverse flow distillation (i.e., to separate flavor compounds and water), for example, preferably under low pressure (less than 300 mbar) and room temperature in order to minimize the thermal load. For an improved extraction yield of water-soluble flavors and antioxidants, it is preferred to carry out the aforementioned wet-grinding step to an average particle diameter of 200 µm or less and further preferably to 100 µm or less, before solid-liquid separation. If necessary, a pasteurisation or sterilisation step may be carried out according to methods known in the art to prevent microorganism spoilage/propagation, e.g. by application of heat, irradiation, chemical sterilisation, and micro-, ultra- or nanofiltration, for example. The pasteurisation process may be carried out in batch or continuous operation, e.g. by the use of HTST (high temperature short time) heat exchangers. In further concentration phase, evaporation of excessive water may performed to obtain a solid composition containing secondary metabolites. Extracted water-soluble flavors and secondary metabolites (e.g. polyphenols) may be then recombined with the above-described extracts from the solid phase to enhance the overall yield of aromas and secondary metabolites.

### In Vitro-Manufactured Extracts and Uses

In a second embodiment, the present invention relates to an *in vitro*-manufactured biomass or secondary metabolite extract obtained by the method according to the first embodiment described above.

As set out above, the cell culture-based biomass may be present in a wet form (i.e. as a slurry) or dried form (i.e. as a powder), depending on whether the biomass may be immediately processed (e.g. in liquid or paste-like food preparations) or is intended to be stored and/or packaged for further use, respectively.

In a third embodiment, the present invention relates to the use of the *in vitro-*manufactured biomass or secondary metabolite extract according to the second embodiment as food, food additive or in cosmetic or pharmaceutical preparations.

Compositions containing the biomass produced according to the methods above may be used in pharmaceutical products, cosmetic products, food products, beverage compositions and can be prepared in accordance with standard techniques well known to those skilled in the art. Pharmaceutical and cosmetic compositions that include the biomass or the secondary metabolite extract described above as an active agent can be suitably formulated at an active dose with an appropriate solid or liquid carrier, depending on the particular mode of administration chosen. Exemplary forms of administration include injectable fluids, topical and oral formulations. Oral formulations can be liquid (for example, syrups, beverages, solutions, or suspensions), or solid (for example, powders, pills, tablets, or capsules). Topical preparations can include ointments, creams, sprays and the like. Formulations suitable for topical administration may be mixed with a solvent, carrier, diluent and/or dispersant.

The *in vitro*-manufactured biomass or secondary metabolite extract according to the second embodiment may be used as a food additive for the purpose of altering the nutritional profile (e.g. enhancing the antioxidant properties of a food composition), enhancing the flavor of foods and/or modifying the texture of food compositions (e.g. as a replacement for flour).

### Food Compositions

In a fourth embodiment, the present invention relates to a food composition, containing a mixture of: *in vitro*-manufactured biomass or secondary metabolite extract according to the second embodiment in an amount of 25% to 99% by weight of the total weight of the food composition, and one or more edible ingredients other than the in vitro-manufactured biomass in an amount of 1% to 75% by weight of the total weight of the food composition.

In order to make ideal use of the advantages of the present invention, the mixture may comprise *in vitro*-manufactured biomass or secondary metabolite extract according to the second embodiment in an amount of 40% to 99% by weight, more preferably in an amount of 50% to 99% by weight, and even further preferably in an amount of 55% to 99% by weight of the total weight of the food composition.

Edible ingredients other than the *in vitro*-manufactured biomass or secondary metabolite extract may be suitably chosen by the skilled artisan dependent on the desired recipe and may be present in a cooked or raw state. In addition, the biomass in the food composition may be subjected to a thermal treatment (e.g. cooking, roasting or baking) before being admixed with the edible ingredients or with the food composition as such, in order to contribute to flavor development via Maillard reactions.

Hybrid food compositions according to the fourth embodiment may take any suitable form and may, for example, be packaged and sold as a paste, in liquid form, as a block (e.g. as a stock or bouillon cube) or a bar, or as a coating or filling, for example.

In a preferred embodiment, the food composition is freshly prepared by admixing wet *in vitro*-manufactured biomass as described above.

As a preferred example of a food composition, which will be described in the examples below, a hybrid guacamole preparation may be mentioned, which may be produced by admixing *in vitro*-manufactured biomass based on endocarp/seed explants of the avocado fruit. However, it will be understood that food compositions according to the present invention are not limited thereto.

The features of the first to fourth embodiments may be freely combined in any combination, except for combinations where at least some of the features are mutually exclusive.

### EXAMPLES

### Establishment of callus and suspension cultures based on avocado fruits (Persea americana)

For callus induction, avocado samples of different ripeness grades (see Table 1) were used as starting material.

**TABLE 1: Avocado samples**

| **Origin** | **Label** | **Ripeness** |
|---|---|---|
| Israel | Qualité & Prix Coop | ripe |
| Israel | Qualité & Prix Coop | unripe |
| Spain | Naturplan Bio Coop | ripe |
| Dominican Republic | not specified | ripe |
| Colombia | not specified | unripe |
| Peru | not specified | unripe |

For the purpose of callus induction, the samples were initially surface-sterilised by cleaning with a brush, immersion in 0.5% sodium hypochlorite (NaClO) solution for 5 minutes and subsequently in 70% (v/v) ethanol for 10 minutes, followed by two washing steps with sterile ultrapure water. Samples from the stone (endocarp), pulp (mesocarp) and peel (exocarp) were cut into explants (0.5 cm x 0.5 cm) using a sterile scalpel and independently used for callus induction. The explants were transferred to solid media with different phytohormone types and concentrations (see Table 2). The closed Petri dishes were incubated at 23 °C, 26 °C, 27°C and 29 °C, respectively, and with (for a period of 16/8 h at 4.4 W m²) and without light exposition. If the induction was successful, the primary callus formed was transferred to new Petri dishes after 4 weeks.

**TABLE 2: Media compositions**

| **Label** | **Basal medium** | **Vitamins** | **Phytohormones** |
|---|---|---|---|
| MS-Avo-1 Medium | MS + 30 g L⁻¹ | MS Vitamins | 2 mg L⁻¹ Indole-3-acetic acid (IAA) |
| | Saccharose | | 0.2 mg L⁻¹ kinetin |
| MS-Avo-2 Medium | MS + 30 g L⁻¹ | MS Vitamins | 2 mg L⁻¹ IAA |
| | Saccharose | | 0.2 mg L⁻¹ kinetin |
| | | | 60 mg L⁻¹ ascorbic acid |
| | | | 40 mg L⁻¹ citric acid |
| MS-Avo-3 Medium | MS + 30 g L⁻¹ | MS Vitamins | 1 mg L⁻¹ 2,4-Dichlorophenoxyacetic acid |
| | Saccharose | | |
| MS-Avo-4 Medium | MS + 30 g L⁻¹ | MS Vitamins | 2 mg L⁻¹ IAA |
| | Saccharose | | 0.2 mg L⁻¹ kinetin |
| | | | 0.1 mg L⁻¹ zeatin |
| MS-Avo-5 Medium | MS + 30 g L⁻¹ | MS Vitamins | 0.5 mg L⁻¹ IAA |
| | Saccharose | | 0.5 mg L⁻¹ IBA |
| | | | 0.2 mg L⁻¹ kinetin |
| | | | 0.5 mg L⁻¹ zeatin |

For mass propagation, calli were transferred to fresh medium (petri dishes) every two to three weeks. In subcultivation, care was taken to ensure that only vital callus was transferred to the new medium. The same media as for callus induction (cf. Table 2) were used for further mass propagation of the callus and the establishment of a suspension culture. As with callus induction, mass propagation was carried out at 23 °C, 26 °C, 27°C and 29 °C, respectively, and under dark conditions or under light exposition (for a period of 16/8 h at 4.4 W m²).

To establish suspension cultures, 2 to 3 g of vital, friable calli were each transferred to a 125 mL shake flask with 30 mL of fresh medium. The suspensions were incubated in darkness in an incubator at 29° C and 120 rpm (50 mm amplitude). After 4, 7, 14 and 21 days, the suspensions were homogenized by transferring the contents of each shake flask into an autoclaved measuring cylinder and only the middle layer (after complete sedimentation) was pipetted into a new shake flask with fresh medium. Once a homogeneous suspension was available, it was subcultured weekly by transferring 30 mL of the suspension with 70 mL of fresh medium into a 500 mL shake flask.

The mass propagation of the suspension cultures was studied in three different bioreactor types, i.e. a cylindrical orbitally shaken bioreactor (TubeSpin^{®} 50 bioreactor, commercially available from TPP), a wave-mixed bioreactor (Biostat^{®} RM Flexsafe 2L SC, commercially available from Sartorius) and a shake flask incubator (Multitron, commercially available from Infors), each operated in batch mode.

Cell growth in the cylindrical orbitally shaken bioreactor (temperature of 29 °C and a shaking rate of 170 rpm (amplitude 50 mm)) was monitored for 4 weeks. Initially, the TubeSpin^{®} 50 bioreactors were inoculated with a working volume of 10 mL and a fresh cell weight of 20 g·L⁻¹. MS Avo 1 medium was used for growth characterization. The entire contents of each bioreactor were harvested weekly and the packed cell volume (PCV), viability, cell fresh weight (CFW), cell dry weight (CFW), total sugar (refractor meter), pH and conductivity was determined (triple determination). In addition, sucrose, fructose, glucose, nitrate, phosphate and ammonium contents were monitored via HPLC.

Growth characterization in the Biostat RM Flexsafe RM 2 L SC (wave-mixed bioreactor with single-use bag with a working volume of 1 L) was carried out in batch mode over 21 days (temperature: 29 °C, tilt rate: 20-25 rpm, tilt angle: 7 °, aeration rate: 0.1 vvm). As the cylindrical orbitally shaken bioreactor, the wave-mixed bioreactor was inoculated with a CFW of 20 g·L⁻¹. The. A 5 to 10 mL sample was withdrawn every two to three days to measure PCV, viability, CFW, CDW, total glucose, pH and conductivity.

As a control batch, a 1 L shake flask with a working volume of 400 mL was used for the shake flask incubator, inoculated with a CFW of 20 g·L⁻¹. A temperature of 29 °C and a shaking rate of 120 rpm (amplitude 50 mm) was adjusted. As with the wave-mixed bioreactor, 5 to 10 mL of sample and the parameters PCV, viability, CFW, CDW, total glucose, pH and conductivity were measured every two to three days. The growth characterization was carried out over 21 days in batch mode.

### Results

No significant influences based on the variety and degree of ripeness of the avocado fruits were identified.

In case of the callus induction from avocado exocarp and mesocarp, a low induction success rate was observed (20% or less). On the other hand, a surprisingly improved induction success rate of at least 50% and typically about 90% was observed for callus induction from avocado pits (endocarp and seed).

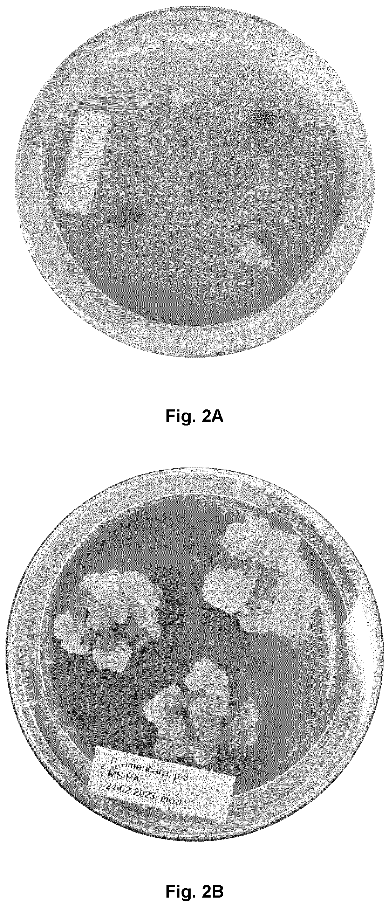

After 2 weeks, all media tested (regardless of variety, degree of maturity and culture conditions) resulted in primary callus. Figure 2A shows an example of the white and friable primary callus formed from the pit of an avocado from the Dominican Republic (MS-Avo-1 medium, dark culture).

After 2 more weeks of cultivation, the primary callus was transferred to fresh MS medium and then passaged every 3 weeks. During the passage and the subsequent mass propagation, care was taken to ensure that only callus with a viability of at least 90% was used. Figure 2B shows the maintenance culture of the callus established from the core of the avocado from Israel (MS-Avo-1 medium, dark culture, 8^{th} passage, grown at 29°C). The doubling time was about 1 week. No differences between the light and dark cultures of the explants were found, while the best growth rates were observed at 29°C.

In Table 3, the callus fresh weights (CFW) and callus dry weights (CDW) at the start and at maximum of the samples grown in the different bioreactor types are shown.

**TABLE 3: Growth rate comparison between different bioreactor types (suspension cells).**

| **Bioreactor type** | **Initial Conditions** | | **Final Conditions** | | |
|---|---|---|---|---|---|
| | CFWₛₜₐᵣₜ [g·L⁻¹] | CDWₛₜₐᵣₜ [g·L⁻¹] | determined after | CFWₘₐₓ [g·L⁻¹] | CDWₘₐₓ [g·L⁻¹] |
| orbitally shaken bioreactor | 22.65 | 1.3 | 28 days | 114.26 | 8.60 |
| wave-mixed bioreactor | 10.80 | 0.76 | 21 days | 139.25 | 13.62 |
| shake flask incubator | 11.04 | 0.78 | 19 days | 67.93 | 6.22 |

As is shown by the above results, the best growth rate was observed with suspension cells in the wave-mixed bioreactor, wherein a maximum CFW of 139.25 g·L⁻¹ was achieved. The biomass productivity achieved in the wave-mixed bioreactor using the specified cultivation parameters was 0.61 g·L⁻¹·d⁻¹, which is more than twice as high as in the shake flask (0.29 g·L⁻¹·d⁻¹) and in the orbitally shaken bioreactor (0.27 g·L⁻¹·d⁻¹).

### Preparation of Guacamole

With the samples obtained in the above-described processes, guacamole was produced by mixing 10 g of the *in vitro*-manufactured avocado cell culture biomass with 10 g spring onions, 2 g sunflower oil, lemon juice, chili pepper, and seasoning with salt and pepper.

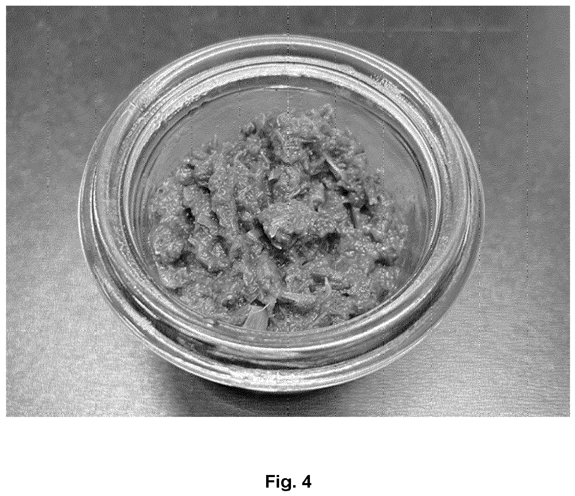

The guacamole samples, one of which is depicted in Fig. 4, were subjected to a taste analysis by an experienced panel. As a result, a pleasantly aromatic and slightly nutty taste profile comparable to that of conventional guacamole was unanimously attributed to the samples.

Accordingly, it was shown that the method of the present invention enables the *in vitro*-production of biomass efficiently and at a scale suitable for the manufacture of food compositions, providing a valuable alternative to conventional fruit processing methods and overcoming the associated disadvantages.

Once given the above disclosure, many other features, modifications, and improvements will become apparent to the skilled artisan.

## Claims

1. Method of processing plant cell cultures based on fruit explants, comprising the steps of:
a) inducing callus from an explant of a fruit selected from drupes, nuts, legumes or berries on callus induction medium and propagating the callus on callus growth medium;
b) selecting a callus clone from the grown callus culture and initiating a suspension cell culture comprising dedifferentiated cells by inoculating the selected callus clone into a suspension cell culture medium;
c) growing fruit suspension cells for a time and under conditions sufficient to produce a suspension including a biomass comprising secondary metabolites; and
d) separating the biomass from the suspension to provide a liquid phase and solid, *in vitro*-manufactured biomass and optionally extracting secondary metabolites from the *in vitro-*manufactured biomass to provide a secondary metabolite extract,
wherein the fruit is selected from and the explant contains endocarp and/or seed material from the drupes, nuts, legumes or berries.

2. The method according to claim 1, wherein the explant contains endocarp and seed material from the drupes, nuts, legumes or berries.

3. The method according to any one of claims 1 or 2, wherein the fruit is selected from berries, and the explant contains endocarp and/or seed material from the berries.

4. The method according to claim 3, wherein the fruit is avocado (*Persea americana*).

5. The method according to any one of claims 1 to 4, wherein step c) is carried out in a bioreactor selected from wave-mixed bioreactor, a stirred tank bioreactor, an orbitally shaken bioreactor, a bubble column bioreactor or an air-lift bioreactor, preferably a wave-mixed bioreactor, a bubble column bioreactor or an air-lift bioreactor.

6. The method of claim 5, wherein hydrodynamic parameters and aeration conditions of the bioreactor are determined based on the concentration of target secondary metabolites present in the biomass.

7. The method according to claim 6, wherein the target secondary metabolites comprise one or more phenolic compounds or polyphenols, preferably phenolic compounds or polyphenols selected from gallic acid, chlorogenic acid, p-hydrobenzoic acid, benzoic acid, coffee acid, catechin, epigallocatechin, glycoside-3-O-kaempferol, quercetin, lutein and rutinoside-3-O-quercetin.

8. The method according to any one of claims 6 or 7, wherein the hydrodynamic parameters and aeration conditions include mixing time, specific power input and/or volumetric oxygen transfer coefficient, the specific power input being adjusted within a range of 100 to 800 W/m³, and the volumetric oxygen transfer coefficient being adjusted within a range of 0.3 to 80 h⁻¹.

9. The method according to any one of claims 1 to 8, wherein the suspension cells are not genetically modified.

10. The method according to any one of claims 1 to 9, wherein the suspension cell culture medium comprises a plurality of mineral salts and optionally plant hormones and/or sucrose.

11. The method according to any one of claims 1 to 10, wherein step c) comprises at least one of:
co-cultivating fruit suspension cells with microorganisms, the microorganisms being preferably selected from one or more species from the group of yeasts, lactic acid bacteria and/or acetic acid bacteria,
adding enzymes, phytohormones or biosynthetic precursors to the suspension; and/or
an elicitation step comprising the addition of one or more abiotic or biotic elicitors to promote biosynthesis of the secondary metabolites.

12. The method according to any one of claims 1 to 11, wherein in step c), growth of the biomass is enhanced by exposing the suspension cell culture to artificial light having a wavelength in the range of 300 to 700 nm and with an intensity sufficient to promote biomass growth and/or by removal of a fraction of the liquid medium during the growth phase.

13. *In vitro-*manufactured biomass or secondary metabolite extract obtained by the method according to any one of claims 1 to 12.

14. Use of the *in vitro-*manufactured biomass according to claim 13 as food, food additive or in cosmetic or pharmaceutical preparations.

15. Food composition, containing a mixture of:
*in vitro*-manufactured biomass or secondary metabolite extract according to claim 13 in an amount of 25% to 99% by weight of the total weight of the food composition, and
one or more edible ingredients other than the *in vitro*-manufactured biomass in an amount of 1% to 75% by weight of the total weight of the food composition.
